# EUROPÄISCHE PATENTANMELDUNG

(11) **EP 4 403 089 A1**
(43) Veröffentlichungstag der Anmeldung: **24.07.2024**
(21) Anmeldenummer: 23152022.2
(22) Anmeldetag: 17.01.2023
(51) Int. Cl.: A61B 1/00, A61B 1/303, A61B 1/32, G06T 7/00

(54) **VISUALISIERUNGSEINRICHTUNG**

(71) Anmelder: Erbe Elektromedizin GmbH, 72072 Tübingen (DE)
(72) Erfinder: Daniel, Yannick, 72336 Balingen (DE); Michelmann, Adrian, 72762 Reutlingen (DE); Jurjut, Ovidiu, 72070 Tuebingen (DE)
(74) Vertreter: Rüger Abel Patentanwälte PartGmbB

(57) **Zusammenfassung**

Die erfindungsgemäße Visualisierungseinrichtung (1) dient insbesondere zur Diagnose und Therapieverfolgung bei der Behandlung von biologischem Gewebe (2). Sie weist eine Kamera (4) und eine daran angeschlossene Bildverarbeitungseinrichtung (5) auf. Diese ist dazu eingerichtet, in den von der Kamera (4) erzeugten Bildern an dem Gewebe (2) vorhandene Referenzstrukturen (7, 8) zu erfassen. Sie ist weiter dazu eingerichtet durch die Untersuchung, beispielsweise in einem Anfärbeversuch, erzeugte optische Indikatoren (9) zu erfassen und zu verfolgen. Die optischen Indikatoren können z.B. angefärbte Flächen sein. Durch die Erfassung und Verfolgung der Strukturen (7, 8) ermittelt die Bildverarbeitungseinrichtung (5) Perspektivveränderungen und erstellt entsprechende Transformationsvorschriften. Mittels dieser trägt die Bildverarbeitungseinrichtung die Indikatoren (9), Repräsentationen (10) und Behandlungspunkte (14) ungeachtet von Lageveränderungen der Kamera (4), des Patienten oder Gewebeverzerrungen am richtigen Ort in das Referenzbild (0, 0', Oⁿ) ein. Die optischen Indikatoren, die behandlungspflichtiges Gewebe kennzeichnen, werden auf diese Weise lagerichtig erfasst und zwar unabhängig von Patientenbewegungen oder Bewegungen des Behandlers.

## Beschreibung

Die Erfindung betrifft eine Einrichtung zur Diagnose und Behandlung von Gewebeveränderungen, insbesondere die Diagnose und Behandlung von zervikalen Neoplasien. Die erfindungsgemäße Visualisierungseinrichtung ist auch in sonstigen ähnlichen Situationen einsetzbar.

Die Behandlung von Zervixgewebe (cervix uteri) kann mit Videounterstützung durchgeführt werden.

Dazu beschreibt der Artikel "Automatic Detection of Anatomical Landmarks in Uterine Cervix Images", Hayit Greenspan, Shiri Gordon, Gali Zimmerman, Shelly Lotenberg, Jose Jeronimo, Sameer Antani, und Rodney Long, IEEE TRANSACTIONS ON MEDICAL IMAGING, VOL. 28, NO. 3, MARCH 2009, die automatische Erkennung von charakteristischen Punkten auf dem Zervixgewebe, um eine webbasierte Datenbank über die Entwicklung von Läsionen aufzubauen zu können, die mit malignen zervikalen Gewebebildungen im Zusammenhang stehen.

Die Merkmalsfilterung und Bildverarbeitung ist aus "KAZE Features Pablo F. Alcantarilla", Adrien Bartoli, und Andrew J. Davison Conference Paper October 2012 DOI: 10.1007/978-3-642-33783-3_16 sowie aus Fast Explicit Diffusion for Accelerated Features in Non-Linear Scale Spaces Pablo Fernandez Alcantarilla Conference Paper September 2013 DOI:10.5244/C.27.13 und "Speeded-Up Robust Features (SURF)", Herbert Bay, Andreas Ess, Tinne Tuyetelaars and Luc Van Gool, ETH Zürich BIWI, Sternwartstraße 7, CH-8092 Zürich, Switzerland preprint submitted to Elsevier vom 10. September 2008 bekannt, siehe auch https://doi.org/10.1016/j.cviu.2007.09.014.

Außerdem sind aus der MY 181157A ein Verfahren und ein System zur Zervix-Positionserkennung mittels Ultraschall bekannt. Zur Zervix-Positionserkennung wird ein Ultraschalltransducer genutzt, der mittels einer mechanischen Führung in X-, Y- und Z-Richtung linear beweglich sowie zusätzlich schwenkbar gelagert ist. Ein automatischer Zervix-Identifikationsalgorithmus erlaubt eine bedienerunabhängige Erfassung der Zervix in Ultraschallbildern.

Außerdem offenbart die DE 10 2019 116 381 A1 ein Verfahren zur Bestimmung der Bildposition eines Markierungspunktes in einem Bild einer Bildsequenz. Dazu wird in einem ersten Bild ein Markierungspunkt festgelegt und eine Transformation zwischen korrespondierenden Teilbereichen des ersten Bildes und einem zweiten Bild der Bildsequenz bestückt. Mit dieser Transformation wird zumindest ein Teilbereich des ersten Bildes transformiert. In dem transformierten Bild wird wiederum der Markierungspunkt lokalisiert und anhand der Transformation in das zweite Bild übertragen.

Bei der Behandlung von Zervixgewebe ist sowohl bei zur Diagnose vorzunehmenden Maßnahmen als auch bei den eigentlichen Behandlungsmaßnahmen damit zu rechnen, dass die Relativposition zwischen Gewebe und Kamera veränderlich ist. Außerdem kann das Zervixgewebe aufgrund der vorgenommenen Manipulation oder auch infolge muskulärer Reaktionen Bewegungen ausführen. Soweit sich eine Behandlung des Gewebes gleichmäßig über die gesamte Gewebefläche erstrecken soll, mag dies unproblematisch sein. Ist jedoch eine Einwirkung lediglich beschränkt auf bestimmte behandlungsbedürftige Bereiche gewünscht, ist es erforderlich, solche Bereiche zu identifizieren und über einen längeren Zeitraum hinweg unabhängig von der Kameraposition oder Gewebebewegungen zu lokalisieren.

Davon ausgehend ist es Aufgabe der Erfindung, eine verbesserte Visualisierungseinrichtung zu schaffen.

Diese Aufgabe wird mit der Visualisierungseinrichtung nach Anspruch 1 gelöst:

Die erfindungsgemäße Visualisierungseinrichtung dient insbesondere zur Diagnose und Therapieverfolgung bei der Behandlung von Zervixgewebe oder anderer biologischer Gewebe, insbesondere Gewebe, das verformbar ist. Die Verformung kann beispielsweise von einer mechanischen Einwirkung bei der Operation oder auch von der Bewegung muskulären Gewebes herrühren, das zu behandeln ist oder das in mechanischer Verbindung zu dem zu behandelnden Gewebe steht.

Zu der Visualisierungseinrichtung gehört mindestens eine Kamera zur Gewinnung mehrerer Bilder oder eines Videostreams während einer Untersuchung des Gewebes. Die Untersuchung kann insbesondere eine Untersuchung sein, die mit einer Veränderung der optischen Erscheinung des Gewebes einhergeht, wie beispielsweise eine Untersuchung mittels eines Anfärbeversuchs. Bei einem solchen wird das zu untersuchende Gewebe mit einer geeigneten auf das Gewebe einwirkenden Flüssigkeit, zum Beispiel Essigsäurelösung oder Lugolsche Lösung, in Berührung gebracht. Darauf folgend bilden sich auf der Gewebeoberfläche Verfärbungen, die Gewebemerkmale charakterisieren. Beispielsweise lassen sich zervikale intraepitheliale Neoplasien auf diese Weise mittels eines Anfärbeversuchs sichtbar machen.

Die zur Aufnahme von Bildern des Anfärbeversuchs oder bei einer sonstigen Gewebeuntersuchung genutzte mindestens eine Kamera kann beispielsweise eine stationär aufgestellte oder auch eine vom Behandler getragene und somit bewegte Kamera sein. Eine bewegte Kamera kann beispielsweise eine Helmkamera, eine in eine VR-Brille des Behandlers integrierte Kamera oder eine sonstige schulter- oder kopfgetragene Kamera sein.

In einer weiteren Ausführungsform kann die Untersuchung auch allein videobasiert und/oder automatisiert erfolgen. So kann beispielsweise eine optional vorhandene Analyseeinheit den Behandlungsbereich allein auf Grundlage der Kamerabilder erkennen oder der Nutzer kann den Behandlungsbereich auf dem Kamerabild manuell definieren, beispielsweise über einen Touchscreen oder eine andere Eingabeeinrichtung. Als optischer Indikator können dann die Koordinaten des definierten Behandlungsgebiets genutzt werden, indem dieser Bildbereich im Kamerabild von der Visualisierungseinrichtung als grafische Repräsentation kenntlich gemacht wird. Dies kann beispielsweise durch eine Begrenzungslinie erfolgen, die die zu behandelnde Fläche umgibt, oder durch eine Einfärbung der zu behandelnden Fläche. Ebenso ist es möglich, dass eine Dosierempfehlung in Form einer Konturkarte oder Falschfarbenkarte zur Kenntlichmachung der zu behandelnden Fläche verwendet wird.

Die von der Kamera gelieferten Bilder bzw. eine Videosequenz wird an eine Bildverarbeitungseinrichtung übertragen, die dazu eingerichtet ist, in den Bildern am Gewebe vorhandene Referenzstrukturen sowie zusätzlich durch die Untersuchung erzeugte oder gewonnene optische Indikatoren zu erfassen und zu verfolgen.

Die Referenzstrukturen können anatomische Strukturen und/oder durch Instrumente gebildete Strukturen sein. Solche Instrumente können z.B. ein Spekulum oder dergleichen sein, die im Bild sichtbar sind. Es können Instrumente sein, mit denen das Operationsfeld offengehalten wird oder die im Operationsfeld angeordnet sind. Die Referenzstrukturen können, insbesondere, wenn es sich dabei um anatomsiche Strukturen handelt, einer zeitlichen Veränderung unterliegen. Beispielsweise kann das Gewebe einer Veränderung durch mechanische Verformung, thermische Beeinflussung, Blutungen, Schwellungen oder dergleichen unterliegen.

Die Bildverarbeitungseinrichtung kann auch dazu eingerichtet sein, grafische Repräsentationen bestimmter, z.B. optischer Indikatoren zu bilden. Die optischen Indikatoren können beispielsweise vom umgebenden Gewebe optisch unterscheidbare Gewebebereiche sein, die während der Untersuchung identifiziert worden sind. Solche optischen Indikatoren können durch grafische Repräsentationen ergänzt oder ersetzt werden. Die grafischen Repräsentationen können die Gewebebereiche umgrenzende Linien, die Gewebebereiche überdeckende Flächen oder dergleichen sein.

Durch die Verfolgung der Strukturen kann die Bildverarbeitungseinrichtung unabhängig von Perspektivwechseln, die sich durch Bewegungen der Kamera oder des Patienten ergeben können, die Relativlage der Kamera in Bezug auf das zu untersuchende Gewebe bestimmen. Die Bildverarbeitungseinrichtung ist weiter dazu eingerichtet, anhand des erkannten Perspektivwechsels die durch die Untersuchung erzeugten optischen Indikatoren lagerichtig in jedes Bild einer Videosequenz einzutragen. Damit können die optischen Indikatoren oder graphische Repräsentationen derselben anhand der Lage der Referenzstrukturen in ein Referenzbild übertragen werden.

Das Referenzbild ist eine Datenrepräsentation des untersuchten Gewebes in einer auf den Raum bezogenen, von der Position der Patienten und der Kamera unabhängigen Position. Das Referenzbild enthält von der Bildverarbeitungseinrichtung identifizierte (statische oder auch sich zeilich ändernde) Referenzstrukturen. Die Bildverarbeitungseinrichtung ist darauf eingerichtet, zu den Bildern einer Bilderfolge anhand der Lage der identifizierten Referenzstrukturen Transformationen zu ermitteln, mittels derer der Inhalt von Bildern der Bilderfolge lagerichtig in das Referenzbild übertragen werden kann. So ist das Referenzbild unabhängig von Bewegungen der Kamera oder des Patienten oder von Gewebeverzerrungen; die Bildverarbeitungseinrichtung rechnet die Bilder einer Bilderfolge oder eines Videos perspektivunabhängig auf das Referenzbild um.

Jedes Bild der Bilderfolge oder des Videos kann durch die Untersuchung erzeugte Indikatoren enthalten, die behandlungsbedürftige Gewebegebiete kennzeichnen. Die Indikatoren sind z.B. farblich unterscheidbare Flächen. Zusätzlich zu den Indikatoren oder an deren Stelle können graphische Repräsentationen, beispielsweise Linien sein, mit denen im Anfärbeversuch oder in einer anderen Untersuchung identifizierte behandlungsbedürftige Gewebepartien eingegrenzt oder anderweitig markiert sind.

Die erfassten Indikatoren oder die graphischen Repräsentationen derselben sind auf das Referenzbild bezogen. Die Bildverarbeitungseinrichtung ist darauf eingerichtet, die Indikatoren oder deren grafische Repräsentationen mittels der anhand der Strukturen ermittelten Transformationsvorschriften in das Referenzbild zu übertragen. Auch kann die Bildverarbeitungseinrichtung darauf eingerichtet sein, die Referenzstrukturen in dem Referenzbild zu aktualisieren, wenn sie einer Veränderung unterliegen. Dazu kann die Bildverarbeitungeeinrichtung dazu eingerichtet sein, anhand der vorhandenen Referenzstrukturen oder anhand anderer Anhaltspunkte, z.B. mittels einer Positionserfassungseinrichtung, einen Perspektivwechsel zu erfassen und zusätzlich eine Veränderung der Form oder des sonstigen Aussehens (z.B. Farb- oder Kontrastveränderung der Referenzstruktur) in das Referenzbild zu übertragen.

Die Bildverarbeitungseinrichtung ist weiter darauf eingerichtet, die Indikatoren oder grafischen Repräsentationen behandlungsbedürftiger Bereiche während der Behandlung des Gewebes aus dem Referenzbild in einen Sichtbereich des Behandlers zu übertragen. Die optischen Indikatoren oder graphischen Repräsentationen derselben werden dabei lagerichtig in dem Sichtbereich des Behandlers übertragen und zwar lagerichtig in Bezug auf die im jeweiligen Live-Bild vorhandenen wiedererkannten Referenzstrukturen. Beispielsweise können die Indikatoren oder graphischen Repräsentationen in eine VR-Brille so eingespiegelt werden, dass sie im Live-Bild, d.h. in dem von dem Behandler wahrgenommenen Bild, an der richtigen Stelle sichtbar werden.

Alternativ können die Indikatoren oder deren graphische Repräsentationen auf einem Monitor einem Leitbild überlagert dargestellt werden. Das Live-Bild kann während der Behandlung von einer stationären oder zum Beispiel schultergestützten Kamera des Behandlers aufgenommen werden. Der Behandler kann den Monitor dann in der Nähe der Patienten aufstellen, um seine Behandlung zu verfolgen oder von Zeit zu Zeit zu überprüfen.

Die erfindungsgemäße Visualisierungseinrichtung kommt ohne direkte Erfassung des Kameraorts, d.h. ohne Kamera-Ortserfassungssystem aus. Es wird ausschließlich mittels der von der Kamera bei der Diagnose sowie von der Kamera bei der Behandlung gelieferten Bilder eine Perspektivanpassung anhand der in den Bildern erfassten Referenzstrukturen vorgenommen.

Die Visualisierungseinrichtung gestattet insbesondere auch die Behandlung und Verfolgung der Behandlung des Zervixgewebes mittels einer Einwirkung, die auf dem Gewebe keine sichtbaren Spuren hinterlässt, d.h., bei der die optische Erscheinung des Gewebes nicht verändert wird. Eine solche Einwirkung kann beispielsweise die Einwirkung auf das Zervixgewebe mittels Licht oder nicht thermischem Plasma, zum Beispiel Kaltplasma, oder anderen Energieformen oder Stoffen sein, wie beispielsweise medikamentöse Einwirkung, Ultraschalleinwirkung oder dergleichen mehr. Die Visualisierungseinrichtung kann dabei dazu eingerichtet sein, den Einwirkungsort bei der medizinischen Behandlung und dessen Bewegung über das Gewebe zu erfassen und als Spur festzuhalten. Die Visualisierungseinrichtung kann weiter dazu eingerichtet sein, diese Spur in einem Monitorbild oder einem anderen Live-Bild beispielsweise in einer VR-Brille sichtbar zu machen. Durch die ständige Perspektivanpassung nicht nur während der Untersuchung, sondern auch während der Behandlung, kann sichergestellt werden, dass der Behandler ausschließlich und in ausreichendem Maße die behandlungsbedürftigen Gewebebereiche behandelt und andere Gewebebereiche schont.

Die bei der Diagnose verwendete Kamera sowie die bei der Behandlung des Gewebes genutzte Kamera können grundsätzlich unterschiedlich ausgebildet sein. Es ist jedoch auch möglich, baugleiche Kameras zu verwenden. Es ist weiter möglich, sowohl für die Untersuchung und Diagnose als auch für die Behandlung ein und dieselbe Kamera, beispielsweise eine schultergestützte Kamera des Behandlers zu nutzen. Diese Kamera kann auch während der Diagnose z.B. mittels einer Stützeinrichtung ortsfest angeordnet und später, während der Behandlung, vom Behandler getragen oder bewegt werden.

Die Bildverarbeitungseinrichtung kann dazu eingerichtet sein, anhand von Veränderung der Lage von anatomischen Strukturen in den Bildern Transformationsvorschriften beispielsweise in Gestalt von Matrizen oder ähnlichem zu ermitteln. Diese Transformationsvorschriften können perspektivische Veränderungen der Bilder und/oder Gewebeverformungen charakterisieren. Perspektivische Veränderungen können sich beispielsweise durch Veränderung der Kameraposition in Bezug auf die Patientin oder Veränderungen der Position der Patientin ergeben. Die zur Perspektivanpassung genutzten Strukturen können insbesondere die Position eines Spekulums, der Rand des Zervixgewebes und/oder die Position anderer anatomischer Strukturen wie zum Beispiel des Zervixkanals oder seiner Mündungsumgebung sein. Selbst bei Verformung oder Verzerrung des Gewebes oder Verlagerung oder Verschwenkung der Kamera in Bezug auf das abgebildete Zervixgewebe lassen sich in der Diagnose erkannte optische Indikatoren lagerichtig in das Live-Bild einkoppeln.

Weitere Einzelheiten vorteilhafter Ausführungsformen der Erfindung sind Gegenstand von Unteransprüchen sowie der Beschreibung und der zugehörigen Zeichnung, In dieser zeigen:
Figur 1 die Zervix und die Bildverarbeitungseinrichtung mit angeschlossener Kamera während der Zervix-Untersuchung, in schematisierter Darstellung,
Figur 2 die Bildverarbeitungseinrichtung mit angeschlossener Bildwiedergabeeinrichtung und Kamera während der Behandlung der Zervix mittels eines Plasmainstruments, in schematisierter Darstellung,
Figur 3 Funktionsblöcke der Bildverarbeitungseinrichtung, in schematisierter Darstellung,
Figur 4 die Bildverarbeitungseinrichtung anhand ihrer Funktionsblöcke zur Perspektivanpassung während der Zervix-Untersuchung,
Figur 5 bis 7 das Referenzbild in seiner Veränderung während der Diagnose,
Figur 8 die Bildverarbeitungseinrichtung anhand wesentlicher Funktionsblöcke bei der Behandlung des Gewebes,
Figur 9 die Bildverarbeitungseinrichtung in einer abgewandelten Ausführungsform anhand wesentlicher Funktionsblöcke bei der Behandlung des Gewebes.

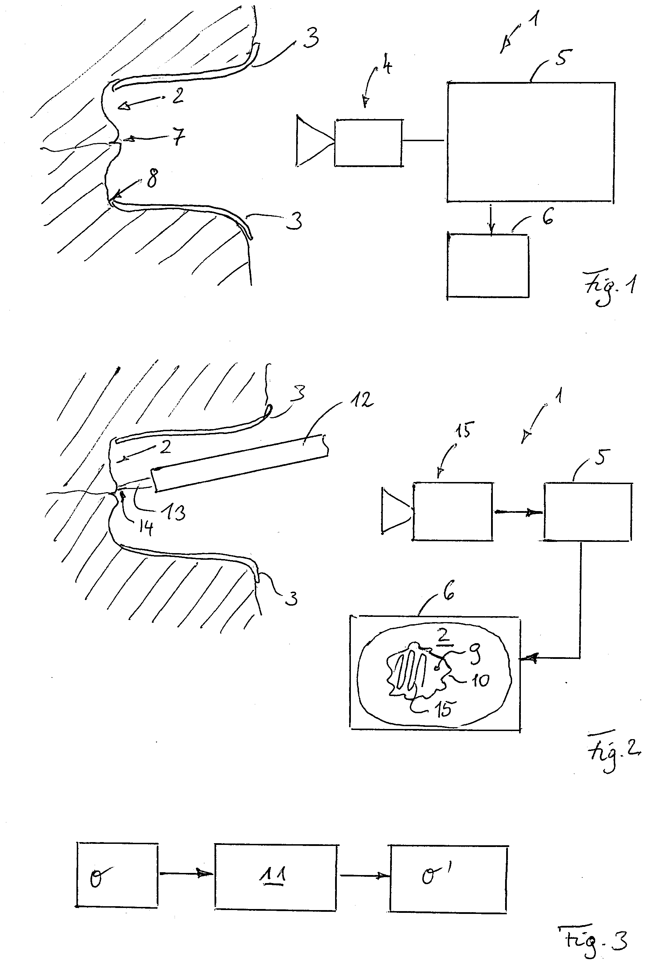

In Figur 1 ist eine Visualisierungseinrichtung 1 bei der Inspektion von biologischem Gewebe, z.B. einer Zervix, veranschaulicht, die über ein Spekulum 3 für eine Kamera 4 sichtbar gehalten ist. Die Kamera 4 ist an eine Bildverarbeitungseinrichtung 5 angeschlossen. Diese kann einen Monitor oder eine sonstige Bildwiedergabeeinrichtung 6 umfassen.

Die Visualisierungseinrichtung 1 dient hier zur Untersuchung von Zervixgewebe, wobei ein ähnliches Setting aber auch bei der Untersuchung anderer Gewebe 2 Anwendung finden kann.

Im vorliegenden Fall beinhaltet die Gewebeuntersuchung einen Anfärbeversuch, bei dem eine geeignete Essenz, beispielsweise Essigsäurelösung oder Lugolsche Lösung, auf das Gewebe 2 aufgebracht wird. Bei Zervixgewebe verfärben sich insbesondere intraepitheliale Neoplasien.

Es sind aber auch Anfärbungen mit Fluoreszenzfarbstoffen und deren Anregung mit UV/VIS-Licht oder die Bestimmung behandlungsbedürftiger Bereiche direkt aus dem Kamerabild mithilfe von Gewebeerkennung durch Einsatz geeigneter Verfahren denkbar.

Die Kamera 4 nimmt während des Anfärbeversuchs Bilder oder eine Videosequenz auf und führt diese der Bildverarbeitungseinrichtung 5 zu. Die Bildverarbeitungseinrichtung 5 dient dabei dazu, in den Bildern der Bilderfolge auftretende Verfärbungen und den zeitlichen Verlauf der Verfärbung und ihres Abklingverhaltens ungeachtet von etwaigen Relativbewegungen zwischen der Kamera 4 und dem Gewebe 2 zu erfassen und dem Gewebe 2 bzw. einem Modell desselben lagerichtig zuzuordnen. Bei der Diagnose, die mehrere Minuten dauern kann, kann sich nicht nur die Position der Kamera 4 in Bezug auf das Gewebe 2 verändern, sondern es kann sich auch das Gewebe 2 beispielsweise durch muskuläre Aktionen verziehen oder verformen.

Die Bildverarbeitungseinrichtung 5 ist dazu eingerichtet beides zu berücksichtigen, nämlich etwaige Verformungen des Gewebes 2, wie auch Relativbewegungen zwischen dem Gewebe 2 und der Kamera 4. Die Bildverarbeitungseinrichtung 5 kann dazu eingerichtet sein, in den Bildern an dem Gewebe 2 vorhandene anatomische Strukturen, wie beispielsweise den Zervixkanal 7 oder den Zervixrand 8, zu erfassen und als Orientierungspunkte und Orientierungsstrukturen für die Perspektivanpassung und Verzerrungsanpassung von Bildern innerhalb der von der Kamera 4 gelieferten Bilderfolge zu nutzen. Es können aber auch nicht-anatomische Strukturen wie der Rand des Spekulums als Orientierungspunkte oder -strukturen genutzt werden.

Zur Erkennung der Strukturen können zweigeteilte Verfahren zur Merkmalserkennung (Feature Detection) und Merkmalsextraktion (Feature Extraction) zum Einsatz kommen, welche beispielsweise auf klassischer Segmentierung und auf Objekterkennung basierend auf Pixeln, Kanten, Regionen, Clustern, Textur, Modell oder Form beruhen. Ebenso kann die Erkennung der Strukturen mit Verfahren des maschinellen Lernens, wie zum Beispiel semantische Segmentierung, sowie durch eine Kombination verschiedener Verfahren erfolgen.

Weiter ist die Bildverarbeitungseinrichtung 5 dazu eingerichtet, bei der Diagnose (das heißt beispielsweise beim Anfärbeversuch) sichtbar gemachte optische Indikatoren 9 (siehe Figur 2) oder grafische Repräsentationen 10 derselben zu erfassen und gegebenenfalls auf der Bildwiedergabeeinrichtung 6 wiederzugeben (siehe dazu Figur 2). Solche Indikatoren 9 können beispielsweise die im Anfärbeversuch erzeugten farblich vom übrigen Gewebe 2 abgesetzten Bereiche sein. Die grafischen Repräsentationen 10 können beispielsweise die mit Kantenfindungsroutinen oder ähnlichen Routinen erzeugten Berandungen der Indikatoren 9 sein, die auf dem Bild der Bildwiedergabeeinrichtungen 6 beispielsweise durch einen Linienzug wiedergegeben werden.

Der Diagnosevorgang ist in Figur 3 schematisch dargestellt. Aus den zu Beginn der Untersuchung aufgenommenen Bildern ergibt sich ein Referenzbild O. In dieses werden mittels eines Transformationsblocks 11 die Indikatoren 9 oder die grafischen Repräsentationen 10 nachfolgend aufgenommener Bilder eingetragen, um ein abgewandeltes Referenzbild O' des untersuchten Gewebes zu erhalten.

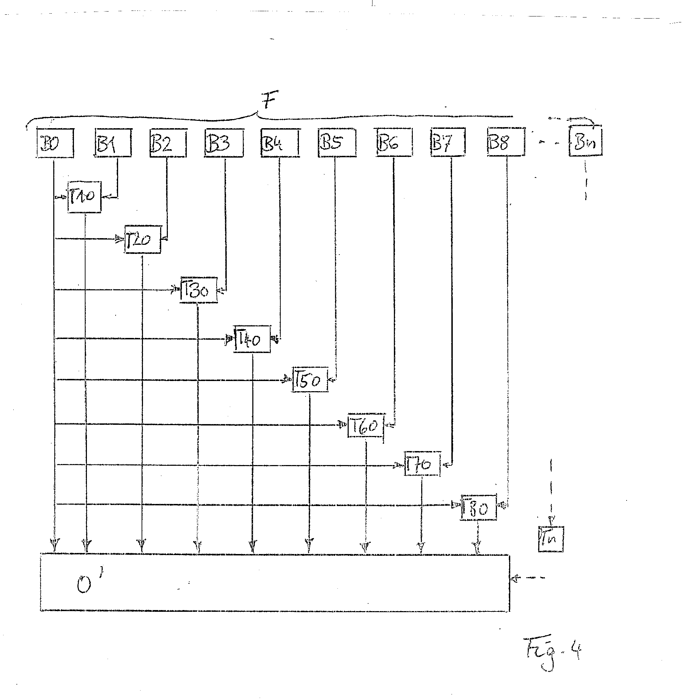

Der Transformationsblock 11 ist Teil der Bildverarbeitungseinrichtung 5 und dient zur lagerichtigen Übertragung der von der Kamera 4 gelieferten Bilderfolge enthaltenen Indikatoren 9. Figur 4 veranschaulicht dazu eine Bilderfolge F, die je nach Ausführungsform einzelne, wenige oder auch hunderte oder tausende von Bildern enthalten kann.

In einem ersten Bild oder aus einem aus den ersten Bildern erzeugten Anfangsbild B0 und einem darauffolgenden Bild B1 wird eine Transformation T10 ermittelt. Das Bild B1 kann gegenüber dem Bild B0 infolge Relativbewegung zwischen dem Gewebe 2 und der Kamera 4 verschoben oder durch Kippung verzogen sein. Zusätzlich kann das Bild B1 eine Verzerrung enthalten, die durch eine Muskelbewegung des Gewebes 2 zustande gekommen ist. Die Bildverarbeitungseinrichtung 5 ermittelt nun insbesondere aus der Lage von Strukturen, die als Orientierungspunkte dienen, sowohl eine Bildverlagerung, als auch eine Bildverzerrung. Die Strukturen können zum Beispiel der Rand 8 des Gewebes 2 oder der Kanal 7 oder ein anderer charakteristischer Punkt des Gewebes 2 sein. Daraus ergibt sich eine Transformation T10. Treten in dem Bild B1 nun beim Anfärbeversuch Farbveränderungen des Gewebes 2 auf, werden diese mittels der Transformation T10 lagerichtig in das Bezugsbild O' übertragen. Ebenso setzt der Vorgang mit Transformationen T20 bis T80, sowie Transformationen für jedes weitere Bild fort.

Das Tracking der anatomischen oder optischen Strukturen kann über pixelbasierte Verfahren wie Methoden basierend auf Phasen-Korrelation und Frequenzbereich, optischer Fluss und Block Matching, oder über merkmalsbasierte Verfahren wie statistische - und Filter-basierte Methoden realisiert werden. Weiterhin kann das Tracking auf Verfahren des maschinellen Lernens, wie beispielweise Object Tracking, oder einer Kombination aus maschinellem Lernen und klassischen Tracking beruhen. Der Begriff "maschinelles Lernen" kann folgendes beinhalten uud ist jedoch nicht beschränkt auf: künstliche neuronale Netzwerke, convolutional neural networks (CNN), recurrrent neural networks (RNN), generative adversarial networks (GAN), Bayes'sche Regression, Naive Bayes, Nächste-Nachbarn-Klassifikation, Support Vector Machine (SVM), neben anderen Techniken aus dem Bereich der Datenanalyse.

Nach Abschluss des Anfärbeversuchs enthält das Referenzbild O' den Indikator 9 und/oder die grafische Repräsentation 10 ungeachtet von Relativbewegungen zwischen Gewebe und Kamera 4 oder Verzerrung des Gewebes 2 lagerichtig.

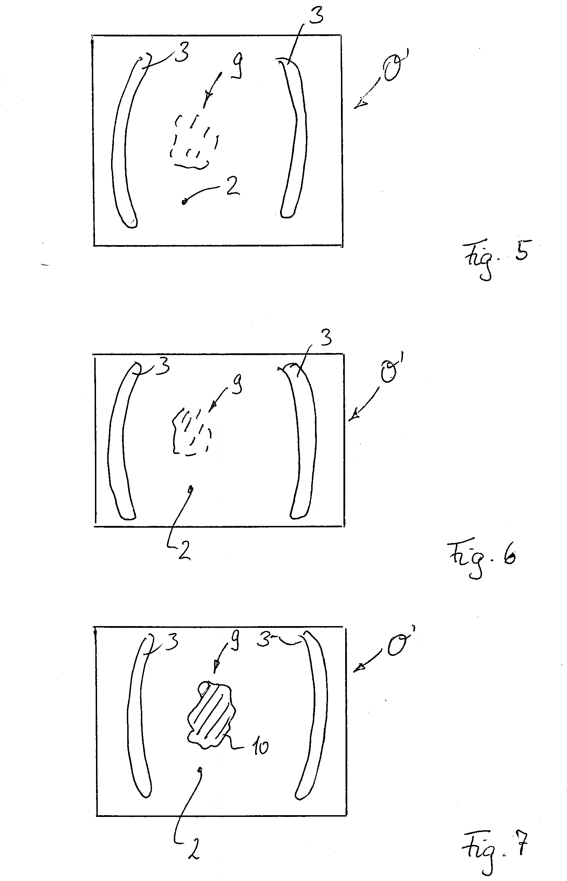

Die Figuren 5 bis 7 veranschaulichen einzelne bei dem Anfärbeversuch anfallende Bilder jeweils lagerichtig übertragen in das Referenzbild O'. In Figur 5 zeigt sich ein im Entstehen begriffener Indikator 9, der im weiteren Verlauf des Anfärbeversuchs immer deutlicher hervortritt, wie die Figuren 6 und 7 veranschaulichen. Infolge der stets stattfindenden Transformation des jeweiligen Bildes hinsichtlich seiner Perspektive und seiner Verzerrung in das Referenzbild O spielen Lageveränderungen während des Anfärbeversuchs keine Rolle.

Die vorbeschriebene Perspektivanpassung kann von der Visualisierungseinrichtung 1 vorzugsweise nicht nur während der Gewebeuntersuchung, sondern darüber hinaus auch bei der Gewebebehandlung durchgeführt werden. Ist beispielsweise während der Untersuchung festgestellt worden, dass mindestens ein behandlungsbedürftiger Gewebebereich existiert, welcher durch den Indikator 9 oder seine grafische Repräsentation 10 angezeigt wird, kann der Behandler chemisch oder physikalisch auf das Gewebe 2 einwirken, um einen Therapieerfolg zu erzielen. Insbesondere kann er dabei eine Einwirkung vornehmen, die auf dem Gewebe 2 keine sichtbaren Spuren hinterlässt. Die Visualisierungseinrichtung 1 kann dabei dazu beitragen die Behandlung auf die von dem Indikator 9 oder der Repräsentation 10 eingegrenzten behandlungsbedürftigen Bereiche zu beschränken und dort im Weiteren eine ausreichende Behandlung herbeizuführen.

Zur Behandlung ist dem Behandler ein Instrument 12 zur Verfügung gestellt, mit dem das Gewebe 2 lokal zu behandeln ist. Beispielsweise kann das Instrument 12 ein Laser, ein Plasmainstrument, insbesondere ein Kaltplasmainstrument, ein Instrument, das einen Stoffstrahl abgibt, oder dergleichen sein.

In dem in Figur 2 veranschaulichten Beispiel erzeugt das Instrument 12 einen Strahl 13 aus einem Kaltplasma. Das Instrument 12 ist vorzugsweise handgeführt. Das Kaltplasma 13 trifft auf einen Punkt 14 und bewegt sich somit mit einer vom Behandler vorgegebenen Geschwindigkeit und auf einem vom Behandler gewählten Weg über das Gewebe 2.

Unabhängig von der Art der Behandlung und des Instruments kann zur Verfolgung der Behandlung eine Kamera 15 vorgesehen sein, die mit der Bildverarbeitungseinrichtung 5 verbunden ist. Bei der Kamera 15 kann es sich um dieselbe Kamera handeln, wie die Kamera 4, die beim Anfärbeversuch genutzt wurde. Es ist aber auch möglich, bei der Untersuchung nach Figur 1 und bei der Behandlung nach Figur 2 unterschiedliche Kameras 4, 15 zu nutzen. Insbesondere die Kamera 15 kann eine kopfgetragene oder schultergeschützte Kamera des Behandlers sein.

Die Bildverarbeitungseinrichtung 5 kann dazu eingerichtet sein, aus der von der Kamera 15 bei der Behandlung gelieferten Bilderfolge und der in den Bildern abgebildeten Position des Instruments 12 den Punkt 14 zu rekonstruieren oder den Punkt 14, wenn das Plasma ausreichend leuchtet, unmittelbar zu bestimmen und in das Referenzbild einzutragen. Auf diese Weise können auf der Bildwiedergabeeinrichtung 6 das Referenzbild O' und die sich durch Beobachtung des Wegs des Punkts 14 über das Gewebe 2 ergebende Spur 15 der Behandlung wiedergegeben werden.

Die Bildwiedergabeeinrichtung 6 kann dabei in der Nähe des Patienten stehen, so dass der Behandler von Zeit zu Zeit auf das Monitorbild schauen und dort seine Behandlung kontrollieren kann. Zur Bildwiedergabe kann der Indikator 9 (oder seine grafische Repräsentation 10) und die Spur 15 jedoch auch auf andere Weise in das Sichtfeld des Behandlers eingebracht werden, beispielsweise durch Wiedergabe in einer VR-Brille, in der dann sowohl der Indikator 9 (oder seine grafische Repräsentation 10) als auch die Spur 15 der Behandlung positionsrichtig in das vom Behandler wahrgenommene reale Bild eingespiegelt werden.

Die Kamera 15 kann beispielsweise eine mit der VR-Brille verbundene Kamera ohne gesonderte Positionserfassung sein. Die Bildverarbeitungseinrichtung 5 gleicht das von der Kamera aufgenommene Bild B1, B2, B3 usw. mit dem Referenzbild O' ab und ermittelt daraus die Transformation T01. Mit dieser Transformation wird der bei der Untersuchung erfasste Indikator 9 (oder seine grafische Repräsentation 10) von dem Referenzbild O' in das erste Bild B1 übertragen.

Findet in dem Bild B1 bereits eine Behandlung statt, kann die Lage des ersten Punkts 14 der Behandlung mittels der Transformation T01 in das Referenzbild O' rückübertragen werden. Entsprechendes gilt für die weiteren Bilder B2, B3 usw. der Bilderfolge, so dass durch die Rückübertragung der Punkte 14 in das Referenzbild O' dort die Behandlungsspur 15 entsteht.

Die Transformationen T01, T02, T03 usw. bewirken dabei eine Perspektiv- und Verzerrungskorrektur, so dass alle Punkte 14 und somit die Spur 15 unverzerrt und lagerichtig in das Referenzbild O' übertragen wird. Dieses wird jedoch durch die Vorwärtstransformationen T01, T02, T03 usw. unabhängig von Kopfbewegungen des Behandlers und unabhängig von Kamerabewegungen gewissermaßen ruhend in dessen Livebild in einer VR-Brille oder, wie in Figur 2 dargestellt, auf einem Monitor oder einer sonstigen Bildwiedergabeeinrichtung 6 wiedergegeben.

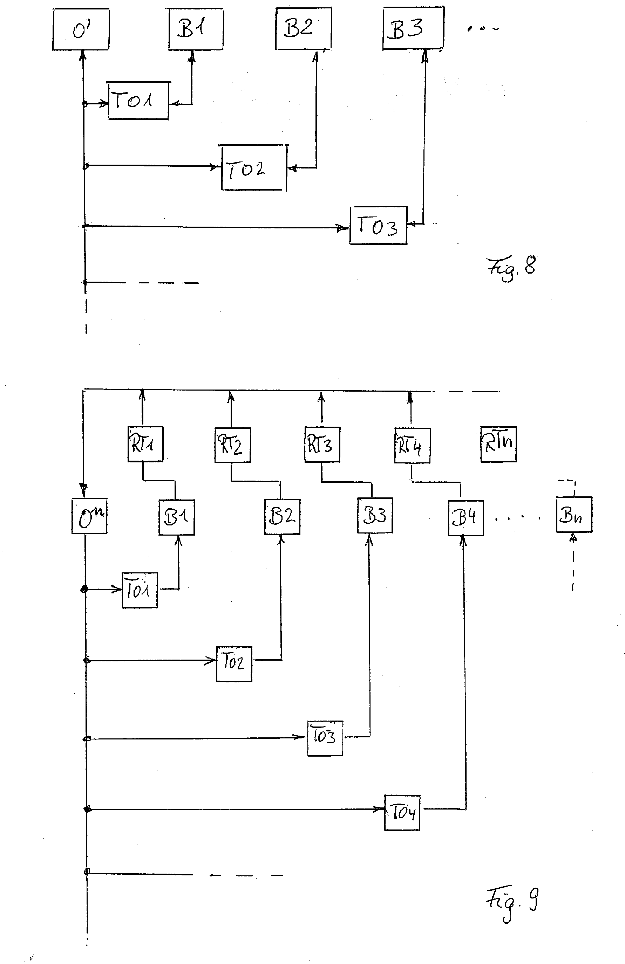

In einer weiteren in Figur 9 veranschaulichten Ausführungsform kann vorgesehen sein, dass das Originalbild O' von Zeit zu Zeit durch ein aktuelleres Referenzbild Oⁿ ersetzt wird und die folgenden Transformationen T01, T02,... auf dieses Referenzbild Oⁿ zurückbezogen werden. Die Substitution des aktuellen Referenzbilds Oⁿ durch ein neues Referenzbild Oⁿ⁺¹ mittels einer Rücktransformation RT1, RT2,... kann entweder durch eine manuelle Eingabe des Nutzers erfolgen oder automatisch nach einem festen Zeitintervall. Ebenso ist es möglich, dass die Übereinstimmung zwischen dem Referenzbild O' oder Oⁿ und dem aktuellen Bild Bn ermittelt wird und bei Unterschreitung einer festgelegten Übereinstimmung die Aktualisierung des Referenzbilds Oⁿ erfolgt. Entsprechend ist es auch möglich, das durch die Rücktransformation RTn bestimmte Ausmaß der Bildveränderung als Kennzeichen für eine nötige Aktualisierung des Referenzbildes Oⁿ zu überwachen. Es kann vorgesehen sein, dass das Referenzbild Oⁿ nur dann aktualisiert wird, wenn das Ausmaß der Bildänderung ein vorgegebenes Maß überschreitet.

Die erfindungsgemäße Visualisierungseinrichtung 1 dient insbesondere zur Diagnose und Therapieverfolgung bei der Behandlung von biologischem Gewebe 2. Es weist eine Kamera 4 und eine daran angeschlossene Bildverarbeitungseinrichtung 5 auf. Diese ist dazu eingerichtet, in den von der Kamera 4 erzeugten Bildern an dem Gewebe 2 vorhandene Referenzstrukturen 7, 8 zu erfassen. Sie ist weiter dazu eingerichtet, durch die Untersuchung, beispielsweise in einem Anfärbeversuch, erzeugte optische Indikatoren 9 zu erfassen und zu verfolgen. Die optischen Indikatoren können z.B. angefärbte Flächen sein. Durch die Erfassung und Verfolgung der Strukturen 7, 8 ermittelt die Bildverarbeitungseinrichtung 5 Perspektivveränderungen und erstellt entsprechende Transformationsvorschriften. Mittels dieser trägt die Bildverarbeitungseinrichtung die Indikatoren 9, Repräsentationen 10 und Behandlungspunkte 14 ungeachtet von Lageveränderungen der Kamera 4, des Patienten oder Gewebeverzerrungen am richtigen Ort in das Referenzbild O, O', Oⁿ ein. Die optischen Indikatoren, die behandlungspflichtiges Gewebe kennzeichnen, werden auf diese Weise lagerichtig erfasst und zwar unabhängig von Patientenbewegungen oder Bewegungen des Behandlers bzw. der Kamera.

Ebenso kann die Bildverarbeitungseinrichtung 5 während der nachfolgenden Behandlung des Gewebes 2 die Spur 15 der Behandlung erfassen und die Spur 15 in das Referenzbild O, O', Oⁿ übernehmen. Dabei nimmt die Bildverarbeitungseinrichtung 5 anhand der als Orientierungspunkte dienenden Referenzstrukturen 7, 8 eine Perspektivanpassung vor, in dem sie beispielsweise durch eine Positionsänderung der Kamera verursachte Bildverzerrungen entzerrt. Die Bildverarbeitungseinrichtung überträgt während der Diagnose die Indikatoren 9 und/oder Repräsentationen 10 anhand der Positionen der Strukturen 7, 8 in das Referenzbild O, O', Oⁿ. Während der Behandlung überträgt die Bildverarbeitungseinrichtung die Indikatoren 9 und/oder Repräsentationen 10 anhand der Lage der Strukturen 7, 8 in das Live-Bild. Die erfindungsgemäße Visualisierungseinrichtung 1 ermöglicht so eine sicherere und komfortablere Behandlung, ohne die Notwendigkeit einer Positionserfassung der Kamera 4, 15 während der Diagnose oder der Behandlung.

### Bezugszeichen:

- 1: Visualisierungseinrichtung
- 2: Gewebe (z.B. cervix uteri)
- 3: Spekulum
- 4: Kamera
- 5: Bildverarbeitungseinrichtung
- 6: Bildwiedergabeeinrichtung
- 7: Cervixkanal
- 8: Cervixrand
- 9: Indikator
- 10: grafische Repräsentation eines Indikators 9
- F: Bilderfolge
- B0 - Bn: Bilder einer Bilderfolge F oder eines Videos
- O: Originalbild
- O': abgewandeltes Originalbild / Referenzbild
- Oⁿ: Referenzbild nach n-ter Aktualisierung
- T10 - Tn0: Transformationen zur Übertragung von Einzelheiten aktueller Bilder in das Referenzbild O, O' oder Oⁿ (bei der Untersuchung)
- T01 - T0n: Transformationen zur Übertragung von Einzelheiten des Referenzbilds O, O' oder Oⁿ in das aktuelle Bild (bei der Behandlung)
- RT1 - RTn: Transformationen zur Übertragung von Einzelheiten aktueller Bilder in das Referenzbild O, O' oder Oⁿ (bei der Behandlung)
- 11: Transformationsblock
- 12: Instrument
- 13: Kaltplasma
- 14: Punkt an dem das Plasma 13 auf das Gewebe 2 trifft
- 15: Spur

## Patentansprüche

1. Visualisierungseinrichtung (1), insbesondere zur Diagnose und Therapieverfolgung bei der Behandlung von Cervixgewebe oder anderem biologischem Gewebe (2),
mit einer Kamera (4) zur Gewinnung mehrerer Bilder (B1, B2 ...) während einer Untersuchung des Gewebes (2),
mit einer Bildverarbeitungseinrichtung (5), die dazu eingerichtet ist, in den Bildern (B1, B2 ...) am Gewebe (2) vorhandene Referenzstrukturen (7, 8) sowie bei der Untersuchung gewonnene optische Indikatoren (9) zu erfassen und/oder zu verfolgen, um die optischen Indikatoren (9) oder grafische Repräsentationen (10) derselben anhand der Lagen der Referenzstrukturen (7, 8) in ein Referenzbild (O, O', Oⁿ) zu übertragen,
um erfasste optische Indikatoren (9) oder grafische Repräsentationen (10) derselben während der Behandlung aus dem Referenzbild (O, O', Oⁿ) in einen Sichtbereich eines Behandlers zu übertragen.

2. Visualisierungseinrichtung nach Anspruch 1, **dadurch gekennzeichnet, dass** die Kamera (4) während der Gewinnung mehrerer Bilder bei der Untersuchung des Gewebes (2) ortsfest angeordnet ist.

3. Visualisierungseinrichtung nach Anspruch 1, **dadurch gekennzeichnet, dass** die Kamera (4) während der Gewinnung mehrerer Bilder bei der Untersuchung des Gewebes (2) beweglich gelagert ist.

4. Visualisierungseinrichtung nach einem der vorstehenden Ansprüche, **dadurch gekennzeichnet, dass** eine Kamera (4, 15) zur Gewinnung mehrerer Bilder (B1, B2 ...) während einer Behandlung des Gewebes (2) vorgesehen ist.

5. Visualisierungseinrichtung nach Anspruch 4, **dadurch gekennzeichnet, dass** die Kamera (15) zur Gewinnung mehrerer Bilder (B1, B2 ...) während einer Behandlung des Gewebes (2) mit der Kamera (4) zur Gewinnung mehrerer Bilder während der Untersuchung des Gewebes (2) identisch ist.

6. Visualisierungseinrichtung nach einem der vorstehenden Ansprüche, **dadurch gekennzeichnet, dass** die Bildverarbeitungseinrichtung (5) dazu eingerichtet ist, anhand von Veränderungen der Lage von Referenzstrukturen (7, 8) in den Bildern (B1, B2 ...) Transformationsvorschriften (T10, T01 ...) zu ermitteln, die perspektivische Veränderungen der Bilder charakterisieren.

7. Visualisierungseinrichtung nach einem der vorstehenden Ansprüche, **dadurch gekennzeichnet, dass** die Bildverarbeitungseinrichtung (5) dazu eingerichtet ist, anhand von Veränderungen der Lage von Referenzstrukturen (7, 8) in den Bildern (B1, B2 ...) Transformationsvorschriften (T10, T01 ...) zu ermitteln, die Gewebeverformungen charakterisieren.

8. Visualisierungseinrichtung nach einem der vorstehenden Ansprüche, **dadurch gekennzeichnet, dass** die Bildverarbeitungseinrichtung (5) dazu eingerichtet ist, Veränderungen der Referenzstrukturen (7, 8) zu erfassen und in das Referenzbild (O, O', Oⁿ) zu übertragen.

9. Visualisierungseinrichtung nach einem der vorstehenden Ansprüche, **dadurch gekennzeichnet, dass** die Bildverarbeitungseinrichtung (5) dazu eingerichtet ist, anhand von Veränderungen der Lage von Referenzstrukturen (7, 8) in den Bildern (B1, B2 ...) Transformationsvorschriften (T10, T01 ...) zu ermitteln, die perspektivische Veränderungen der Bilder (B1, B2 ...) und Gewebeverformungen charakterisieren.

10. Visualisierungseinrichtung nach einem der vorstehenden Ansprüche, **dadurch gekennzeichnet, dass** die Bildverarbeitungseinrichtung (5) dazu eingerichtet ist, bei der Behandlung anhand der Lage der am Gewebe (2) vorhandene Referenzstrukturen (7, 8) die durch die Untersuchung erzeugten optischen Indikatoren (9) oder grafische Repräsentationen (10) derselben aus dem Referenzbild (O, O', Oⁿ) in ein vom Behandler optisch erfassbares Bild zu übertragen.

11. Visualisierungseinrichtung nach einem der vorstehenden Ansprüche, **dadurch gekennzeichnet, dass** die Bildverarbeitungseinrichtung (5) dazu eingerichtet ist, die durch die Untersuchung erzeugten optischen Indikatoren (9) oder deren grafische Repräsentationen (10) aus dem Referenzbild (O, O', Oⁿ) auf einer Bildwiedergabeeinrichtung (6) in Überlagerung mit Bildern (B1, B2 ...) wiederzugeben, die während der Behandlung aufgenommen werden.

12. Visualisierungseinrichtung nach einem der vorstehenden Ansprüche, **dadurch gekennzeichnet, dass** die Bildverarbeitungseinrichtung (5) dazu eingerichtet ist, die durch die Untersuchung erzeugten optischen Indikatoren (9) oder deren grafische Repräsentationen (10) aus dem Referenzbild (O, O', Oⁿ) in eine VR-Brille zur Überlagerung der vom Behandler sichtbaren realen Bilder auszugeben, wobei die VR-Brille eine Kamera (15) aufweisen kann, die an die Bildverarbeitungseinrichtung (5) angeschlossen ist.

13. Visualisierungseinrichtung nach einem der vorstehenden Ansprüche, **dadurch gekennzeichnet, dass** die Bildverarbeitungseinrichtung (5) dazu eingerichtet ist, während der Behandlung eine Spur (15) der Einwirkung eines Instruments (12) auf das Gewebe (2) aufzuzeichnen.

14. Visualisierungseinrichtung nach Anspruch 13, **dadurch gekennzeichnet, dass** die Bildverarbeitungseinrichtung (5) dazu eingerichtet ist, die während der Einwirkung des Instruments (12) auf das Gewebe (2) aufgezeichnete Spur (15) in den Sichtbereich eines Behandlers zu übertragen.

15. Visualisierungseinrichtung nach Anspruch 14, **dadurch gekennzeichnet, dass** die Bildverarbeitungseinrichtung (5) dazu eingerichtet ist, die während der Einwirkung des Instruments (12) auf das Gewebe (2) aufgezeichnete Spur (15) entsprechend der vom Behandler eingenommenen Perspektive und/oder entsprechend einer Gewebeverformung in den Sichtbereich eines Behandlers zu übertragen.
